# EUROPEAN PATENT SPECIFICATION

(11) **EP 2 901 938 B1**
(45) Date of publication and mention of the grant of the patent: **20.03.2019**
(21) Application number: 14176134.6
(22) Date of filing: 08.07.2014
(51) Int. Cl.: A61B 8/00

(54) **Ultrasonic probe and ultrasonic diagnostic apparatus including the same**
Ultraschallsonde und Ultraschalldiagnosevorrichtung damit
Sonde ultrasonique et dispositif et appareil ultrasonique l'incluant

(30) Priority: 29.01.2014 KR 20140011526
(43) Date of publication of application: 05.08.2015
(73) Proprietor: Samsung Medison Co., Ltd., Gangwon-do 250-870 (KR)
(72) Inventor: Min, Hae-kee, Gangwon-do (KR)
(74) Representative: Schmid, Wolfgang

(56) References cited:
- KR-A- 20130 132 171
- US-A- 4 149 419
- US-A- 4 231 373
- US-A- 4 466 444
- US-A- 4 895 158

## Description

### BACKGROUND

### 1. Field

One or more embodiments of the present invention relate to an ultrasonic probe used for ultrasonic diagnoses and an ultrasonic diagnostic apparatus including the same.

### 2. Description of the Related Art

An ultrasonic diagnostic apparatus is an apparatus for diagnosing a target object by visualizing internal body parts of the target object, and acquires an image of a certain part from among the internal body parts of the target object by transmitting ultrasonic signals to the target object and receiving information via echo signals reflected from the target object.

In comparison with a diagnostic apparatus using X rays, the ultrasonic diagnostic apparatus has high stability, is capable of displaying an image on a screen in real time, and there is no possibility of radiation exposure, and thus the ultrasonic diagnostic apparatus is widely used along with other image diagnostic apparatuses.

An ultrasonic diagnostic apparatus has a probe which contacts a target object in order to examine internal body parts thereof. The probe includes an ultrasonic module which generates ultrasonic signals and transmits/receives the ultrasonic signals to/from the target object. According to various arrangements of piezoelectric vibrators forming the ultrasonic module, types of the ultrasonic probe may be classified into a linear type, a convex type, a circular type, etc. Also, the ultrasonic probe may be classified into an ultrasonic probe for acquiring a two-dimensional (2D) image and an ultrasonic probe for acquiring a three-dimensional (3D) image according to the types of the acquired images.

The ultrasonic probe for acquiring a 3D image may have a module-driving structure formed to acquire an image by reciprocating motion of an ultrasonic module within a predetermined range.

However, in the above case, because of reciprocating motion of the ultrasonic module, it is necessary to reduce movement velocity when the ultrasonic module changes its movement direction. Such velocity reduction may decrease an image processing speed of the ultrasonic diagnostic apparatus. Also, vibration and noises may be generated while the ultrasonic module changes its movement direction. US 4 149 419 A discloses an ultrasonic probe with a rotating motion of the ultrasonic module.

### SUMMARY

The present invention is as defined in the appended claims.

One or more embodiments of the present invention include an ultrasonic probe including an ultrasonic module capable of rotating at least about 360° degrees in one direction, and an ultrasonic diagnostic apparatus including the ultrasonic probe.

Additional aspects will be set forth in part in the description which follows and, in part, will be apparent from the description, or may be learned by practice of the presented embodiments.

According to one or more embodiments of the present invention, an ultrasonic probe includes: a housing; an ultrasonic module arranged inside the housing; at least one signal line which transmits/receives signals to/from the ultrasonic module; a support barrel which is supported by the housing, in which the at least one signal line is arranged and an outer surface of which has at least one ring electrode which electrically connects the at least one signal line to the ultrasonic module; and a rotation barrel which is rotatable around the support barrel in one direction and supports the ultrasonic module so as to maintain an electrical contact of the ultrasonic module to the at least one ring electrode.

The ultrasonic module may be formed to be rotatable at least about 360° in one direction due to the rotation barrel.

The at least one ring electrode may be continuously formed along a circumference direction of the support barrel.

The at least one ring electrode may be a plurality of ring electrodes, and the plurality of ring electrodes may be arranged to be separated from each other along a lengthwise direction of the support barrel.

The ultrasonic module may further include contact electrodes protruding towards the support barrel from an inside of the rotation barrel.

The contact electrodes may be bent due to a contact with the at least one ring electrode.

The ultrasonic probe may further include a driving unit which rotates the rotation barrel.

The ultrasonic module may rotate about a rotation axis which is perpendicular to a lengthwise direction of the housing.

The ultrasonic module may further include a controller which makes the ultrasonic module transmit or receive ultrasonic signals at some of regions where the ultrasonic module rotates while the ultrasonic module rotates.

The ultrasonic module may rotate about a rotation axis which is parallel to lengthwise direction of the housing.

According to one or more embodiments of the present invention, an ultrasonic diagnostic apparatus includes: an ultrasonic probe which transmits ultrasonic signals to a target object and receives echo signals reflected from the target object; an image processor which generates an ultrasonic image based on the received echo signal; and a display unit which displays the generated ultrasonic image. The ultrasonic probe includes: a housing; an ultrasonic module arranged inside the housing; at least one signal line which transmits/receives signals to/from the ultrasonic module; a support barrel which is supported by the housing, in which the at least one signal line is arranged, and an outer surface of which has at least one ring electrode which electrically connects the at least one signal line to the ultrasonic; and a rotation barrel which is rotatable around the support barrel in one direction and supports the ultrasonic module so as to maintain an electrical contact of the ultrasonic module to the at least one ring electrode.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram of a structure of an ultrasonic diagnostic apparatus according to an embodiment of the present invention;
FIG. 2 is a schematic view of a structure of an ultrasonic probe according to an embodiment of the present invention;
FIG. 3 is a schematic view of a structure of an ultrasonic probe according to another embodiment of the present invention;
FIG. 4 is an exploded perspective view of the ultrasonic probe of FIG. 2;
FIG. 5 is an exploded perspective view showing some parts of FIG.4;
FIG. 6A is a cross-sectional view of an ultrasonic module of FIG. 4, taken along a line VI-VI of FIG. 4;
FIGS. 6B and 6C are cross-sectional views sequentially showing one-way rotation states of the ultrasonic module of FIG. 6A in one direction; and
FIG. 7 is a cross-sectional view of an ultrasonic probe according to another embodiment of the present invention.

### DETAILED DESCRIPTION

In the description of the present invention, since terms used herein are defined in consideration of the functions of the present invention and commonly-used terms, they may vary according to users' intentions, practices or the advent of new technologies. In addition, terms may be arbitrarily determined by an applicant and, in this case, they may be later described in detail. Therefore, the terms used herein must be defined based on meanings thereof and contents of the entire specification.

Throughout the specification, when a portion "includes" an element, another element may be further included, rather than excluding the existence of the other element, unless otherwise described. Also, throughout the specification, the terms such as "unit" and "module" refer a unit which processes at least one function or operation, and may be configured as hardware, software, or a combination thereof.

Throughout the specification, an "ultrasonic image" denotes an image of a target object acquired by using ultrasonic waves. Also, the target object may include a person or an animal, or some parts of a person or an animal. For example, the target object may include organs or blood vessels included in a liver, a heart, a uterus, a brain, breasts or an abdomen. Also, the target object may include a phantom, and the phantom may refer a material having a volume which approximates to a density of an organism or an effective atomic number.

Also, throughout the specification, the term "user" may be a medical expert such as a doctor, a nurse, a medical technologist and a medical imaging technologist, or a technician who fixes medical equipment, but is not limited thereto.

Reference will now be made in detail to embodiments, examples of which are illustrated in the accompanying drawings. Expressions such as "at least one of," when preceding a list of elements, modify the entire list of elements and do not modify the individual elements of the list.

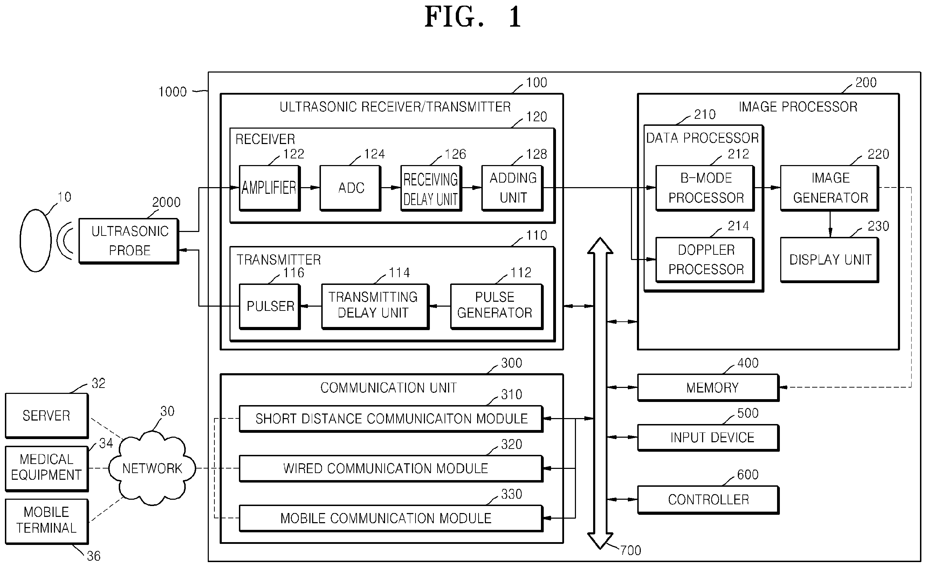

FIG. 1 is a block diagram of a structure of an ultrasonic diagnostic apparatus 1000 according to an embodiment of the present invention. The ultrasonic diagnostic apparatus 1000 may include an ultrasonic probe 2000, an ultrasonic transmitter/receiver 100, an image processor 200, a communication unit 300, a memory 400, an input device 500, and a controller 600, and each of the above-listed components may be connected via a bus 700.

The ultrasonic diagnostic apparatus 1000 may be configured as a cart-type or portable ultrasonic diagnostic apparatus. Examples of the portable ultrasonic diagnostic apparatus may include a picture archiving and communication system (PACS) viewer, a smart phone, a laptop computer, a personal digital assistant (PDA), or the like, but are not limited thereto.

The ultrasonic probe 2000 transmits ultrasonic signals to a target object 10 and receives echo signals reflected from the target object 10 according to driving signals transmitted by the ultrasonic transmitter/receiver 100. The ultrasonic probe 2000 includes transducers (not shown), and each of the transducers vibrates according to the driving signals and generates ultrasonic waves which are sound energy. Also, the ultrasonic probe 2000 may be connected to a main body of the ultrasonic diagnostic apparatus 1000 via a wired or wireless method, and the ultrasonic diagnostic apparatus 1000 may include a plurality of ultrasonic probes 2000 according to configuration types.

The transmitter 110 provides driving signals to the ultrasonic probe 2000 and includes a pulse generator 112, a transmitting delay unit 114 and a pulser 116. The pulse generator 112 generates pulses for generating transmission ultrasonic waves according to a predetermined pulse repetition frequency (PRF), and the transmitting delay unit 114 applies a delay time for determining transmission directivity to the pulses. Each pulse to which the delay time is applied corresponds to one of piezoelectric vibrators included in the ultrasonic probe 2000. The pulser 116 is a timing corresponding to each pulse to which the delay time is applied, and transmits driving signals (or driving pulses) to the ultrasonic probe 2000.

The receiver 120 generates ultrasonic data by processing echo signals transmitted by the ultrasonic probe 2000, and may include an amplifier 122, an analog digital converter (ADC) 124, a receiving delay unit 126 and an adding unit 128. The amplifier 122 amplifies the echo signals in each of channels, and the ADC 124 AD-converts the amplified echo signals. The receiving delay unit 126 applies a delay time for determining receipt directivity to each signal which is digitally converted, and the adding unit 128 generates ultrasonic data by adding echo signals processed by the receiving delay unit 126. The receiver 120 may not include the amplifier 122, depending on configuration types. That is, when a sensitivity of the ultrasonic probe 2000 is improved or the number of bits to be processed by the ADC 124 is increased, the amplifier 122 may not be included.

The image processor 200 generates an ultrasonic image via a scan conversion process with regard to the ultrasonic data generated in the ultrasonic transmitter/receiver 100, and displays the generated ultrasonic image. The ultrasonic image may include a grayscale image, which is acquired by scanning the target object 10 in an amplitude (A) mode, a brightness (B) mode and a motion (M) mode, as well as a Doppler image, which shows the target object 10 which moves using a Doppler effect. The Doppler image may include a blood flow Doppler image (or a color Doppler image) which shows a flow of blood, a tissue Doppler image which shows movements of tissues and a spectral Doppler image which marks movement velocity of the target object 10 with a wavy form.

The B-mode processor 212 extracts B-mode components from the ultrasonic data and processes the extracted B-mode components. The image generator 220 may generate an ultrasonic image which marks an intensity of a signal with brightness based on the B-mode components extracted by the B-mode processor 212.

Likewise, the Doppler processor 214 extracts Doppler components from the ultrasonic data, and the image generator 220 may generate a Doppler image which marks movements of the target object 10 with a color or a wavy form based on the extracted Doppler components.

The image generator 220 may generate a three-dimensional (3D) image via a volume rendering process with regard to volume data or an elasticity image which visualizes a degree of deformity of the target object 10. Furthermore, the image generator 220 may display a variety of additional information as texts or graphics on the ultrasonic image. The generated ultrasonic image may be stored in the memory 400.

The display unit 230 may display the generated ultrasonic image. The display unit 230 may display the ultrasonic image and a variety of information processed in the ultrasonic diagnostic apparatus 1000 on a screen via a graphic user interface (GUI). The ultrasonic diagnostic apparatus 1000 may include two or more display units 230, depending on configuration types.

The communication unit 300 has a wired or wireless connection with a network 30 and communicates with an external device or a server. The communication unit 300 may share data with a server 32 in a hospital or another medical apparatus 34 in the hospital which is connected to the communication unit 300 via a picture archiving and communication system (PACS). Also, the communication unit 300 may perform data communication according to digital imaging and communications in medicine (DICOM) standards.

The communication unit 300 may receive/transmit data related to a diagnosis of the target object 10, for example, an ultrasonic image, ultrasonic data, Doppler data, or the like via the network 30. Also, the communication unit 300 may receive/transmit images acquired by other medical apparatuses such as a computed tomography (CT) apparatus, a magnetic resonance imaging (MRI) apparatus and an X-ray apparatus. Furthermore, the communication unit 300 may receive information with regard to a diagnostic history, treatment schedules, etc. of the target object 10 and may use the information to diagnose the target object 10. In addition, the communication unit 300 may perform data communication with not only the server 32 or the medical apparatus 34 in the hospital but also a mobile terminal 36 of a doctor or a patient.

The communication unit 300 has a wired or wireless connection with the network 30 and may share data with the server 32, the medical apparatus 34 or the mobile terminal 36. The communication unit 300 may include one or more components which enables communication with an external device, and may include, for example, a short distance communication module 310, a wired communication module 320 and a mobile communication module 330.

The short distance communication module 310 is a module for short distance communication within a predetermined distance. Short distance communication technologies may include, but are not limited to, wireless LAN, Wi-Fi, Bluetooth, Zigbee, Wi-Fi Direct (WFD), ultra wideband (UWB), an infrared data association (IrDA), Bluetooth low energy (BLE), near field communication (NFC), etc.

The wired communication module 320 is a module for communication using electrical signals or optical signals, and wired communication technologies may include a pair cable, a coaxial cable, a fiber optic cable, Ethernet cable, etc.

The mobile communication module 330 receives/transmits wireless signals from/to at least one of a base station, an external terminal and a server. The wireless signals may include various forms of data according to receipt/transmission of voice call signals, video call signals or text/multimedia messages.

The memory 400 stores a variety of information processed in the ultrasonic diagnostic apparatus 1000. For example, the memory 400 may store medical data with regard to a diagnosis of the target object 10, for example, input/output ultrasonic data, an ultrasonic image, etc., and may also store algorithms or programs that may be executed in the ultrasonic diagnostic apparatus 1000.

The memory 400 may be configured as various storage media such as a flash memory, a hard disk and an electrically erasable and programmable random access memory (EEPROM). Also, the ultrasonic diagnostic apparatus 1000 may operate web storage or a cloud server which performs a storage function of the memory 400 on the Web.

The input device 500 denotes a medium for receiving, from a user, data which controls the ultrasonic diagnostic apparatus 1000. The input device 500 may include, but is not limited to, hardware components such as a key pad, a mouse, a touch panel, a touch screen, a track ball and a jog switch, and may further include various input media such as an electrocardiogram measurement module, a breath measurement module, a voice recognition sensor, a gesture recognition sensor, a fingerprint recognition sensor, an iris recognition sensor, a depth sensor and distance sensor.

The controller 600 generally controls operations of the ultrasonic diagnostic apparatus 1000. That is, the controller 600 may control operations among the ultrasonic probe 2000, the ultrasonic receiver/transmitter 100, the image processor 200, the communication unit 300, the memory 400 and the input device 500 of FIG. 1.

Some or all of the ultrasonic probe 2000, the ultrasonic receiver/transmitter 100, the image processor 200, the communication unit 300, the memory 400, the input device 500 and the controller 600 may operate as a software module, but the operations of some or all of the above-listed components are not limited thereto. Also, Some of the ultrasonic probe 2000, the ultrasonic receiver/transmitter 100, the image processor 200, the communication unit 300, the memory 400, the input device 500 and the controller 600 may operate as hardware. At least some of the ultrasonic receiver/transmitter 100, the image processor 200, the communication unit 300 may be included in the controller 600, but are not limited thereto.

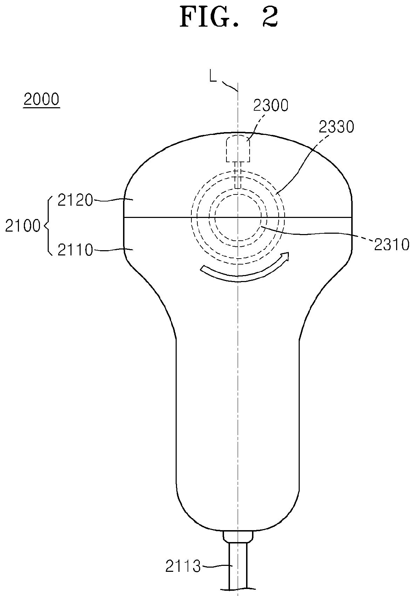

FIG. 2 is a schematic view of a structure of the ultrasonic probe 2000 according to an embodiment of the present invention. The ultrasonic probe 2000 includes a housing 2100 and an ultrasonic module 2300 arranged to rotate in the housing 2100.

The housing 2100 includes a handle case 2110 and a cap 2120 which is arranged at a front end of the handle case 2110 and contacts the target object 10 to be examined. The handle case 2110 fixes the cap 2120 and may have a shape which allows the user to easily grasp the handle case 2110. The cap 2120 transmits the ultrasonic signals, which are emitted from the ultrasonic module 2300 arranged in the housing 2100, to the target object 10 of FIG. 1 and may function to transmit the echo signals reflected from the target object 10 to the ultrasonic module 2300.

The ultrasonic module 2300 includes at least one transducer (not shown) and may be configured to rotate at least about 360° in one direction. That is, the ultrasonic module 2300 does not repeatedly move within a predetermined range of degrees, but may have a structure capable of rotating about 360° and more in one direction. The at least one transducer may include a piezoelectric vibrator, a sound absorption layer, an acoustic matching layer, etc. The housing 2100 includes a space where the ultrasonic module 2300 may rotate about 360° and more in one direction.

Since the ultrasonic module 2300 rotates in one direction, periodical sections for reducing movement velocity of the ultrasonic module 2300 which may appear during reciprocating motion may be removed. Thus, a decrease in the movement velocity of the ultrasonic module 2300 may be prevented. Also, vibration and noises, which are generated while the movement velocity of the ultrasonic module 2300, may be reduced.

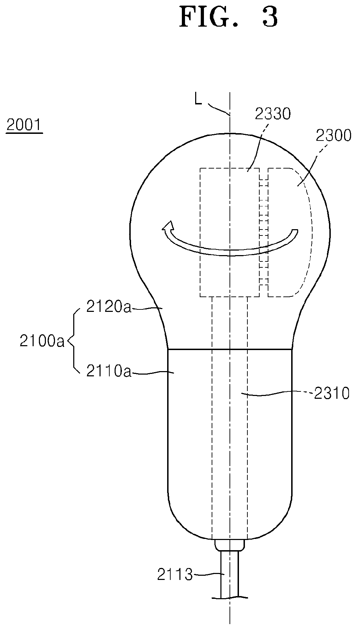

The ultrasonic module 2300 rotates about a rotation axis. The rotation axis has a direction crossing a lengthwise direction (L) of the housing 2100, for example, a vertical direction. However, the rotation axis of the ultrasonic module 2300 is not limited thereto, and may vary according to necessity. For example, as shown in FIG. 3, the ultrasonic module may rotate about a rotation axis which is parallel to the lengthwise direction (L) of the housing 2100. In this regard, structures of the handle case 2110a and the cap 2120a of FIG. 3 may be partially different from the handle case 2110 and the cap 2120 of Fig. 2.

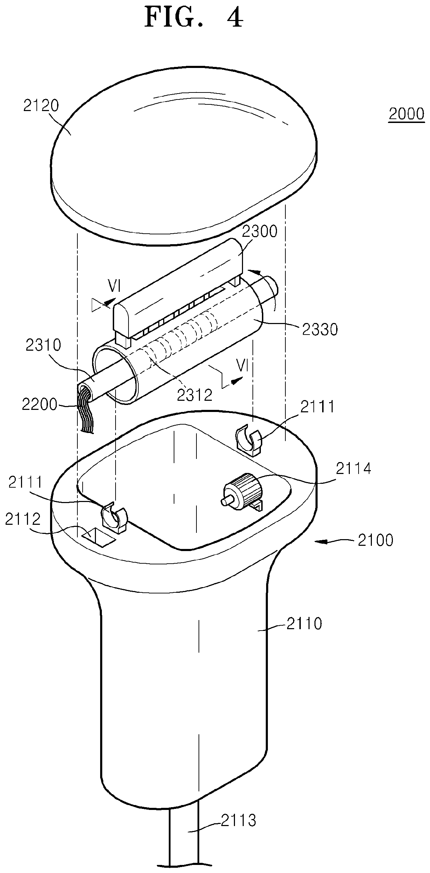

For transmission and receipt of the ultrasonic signals, the ultrasonic module 2300 needs to be connected to signal lines 2200 (refer to FIG. 4) which receives/transmits electrical signals from/to the receiver 120/the transmitter 110. However, when the ultrasonic module 2300 rotates about 360° and more in one direction in a state where the signal lines 2200 are directly connected to the ultrasonic module 2300, the signal lines 2200 connected to the rotating ultrasonic module 2300 are twisted due to a rotation of the ultrasonic module 2300. Also, when there are signal lines 2200, the signal lines 2200 may be twisted around each other, and twists of the signal lines 2200 may cause damage to the signal lines 2200 or to portions where the signal lines 2200 are connected to other components.

However, in the present embodiment, although the ultrasonic module 2300 rotates in one direction, a structure of connecting the ultrasonic module 2300 to the signal lines 2200, which may prevent torsion or the twists of the signal lines 2200, is provided.

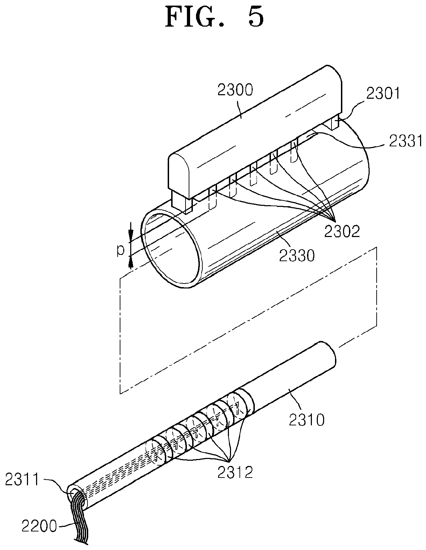

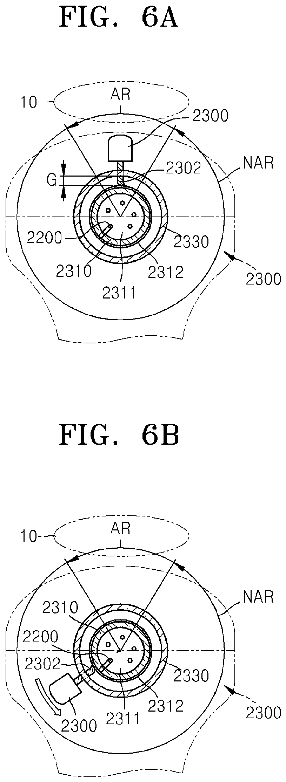

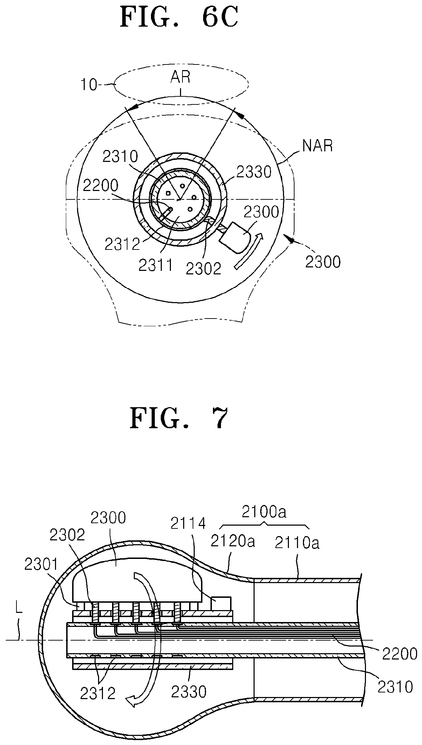

FIG. 4 is an exploded perspective view of the ultrasonic probe 2000 of FIG. 2, and FIG. 5 is an exploded perspective view showing some parts of the ultrasonic probe 2000 of FIG.4. FIG. 6A is a cross-sectional view of the ultrasonic module 2300 of FIG. 4, taken along a line VI-VI of FIG. 4, and FIGS. 6B and 6C are cross-sectional views sequentially showing one-way rotation states of the ultrasonic module 2300 of FIG. 6A in one direction.

Referring to FIGS. 4 and 5, there are the signal lines 2200, a support barrel 2310 in which the signal lines 2200 are arranged, a rotation barrel 2330 capable of rotating around the support barrel 2310, and the ultrasonic module 2300 attached to the rotation barrel 2330.

Both end portions of the support barrel 2310 are supported by support members 2111 formed on the handle case 2110. A cavity 2311 into which the signal lines 2200 may be inserted is formed in the support barrel 2310. Some part of each of the signal lines 2200 are inserted into the support barrel 2310 and other part of each of the signal lines 2200 may be connected to a connection cable 2113 by passing through an opening 2112 formed in the handle case 2110. Through the connection cable 2113, the signal lines 2200 may be connected to the transmitter 110 and the receiver 120 arranged on the main body of the ultrasonic diagnostic apparatus 1000.

Ring electrodes 2312 are arranged on an outer surface of the support barrel 2310. For example, the ring electrodes 2312 may be arranged to be separated from each other in a lengthwise direction of the support barrel 2310. The ring electrodes 2312 are connected to the signal lines 2200 inserted into the cavity 2311, respectively. The number of the ring electrodes 2312 may correspond to that of the signal lines 2200. For example, as shown in FIGS. 4 and 5, when there are five ring electrodes 2312, there may be five signal lines 2200. As another example, although not illustrated in the drawings, when there are a hundred and ninety two (192) ring electrodes 2312, the number of the signal lines 2200 may also be 192.

Each of the ring electrodes 2312 is continuously formed along a circumference direction of the support barrel 2310. Accordingly, the ring electrodes 2312 may remain in electrical contact with the ultrasonic module 2300 even though the ultrasonic module 2300 rotates in one direction. Materials of the ring electrodes 2312 may include an electrically conductive material so as to transmit electrical signals between the ultrasonic module 2300 and the signal lines 2200. A conductive material may be a metal such as copper, iron, aluminum, nickel, gold and silver. However, the conductive material is not limited thereto, and the ring electrodes 2312 may be formed of a non-conductive material which may be generally used to transmit electrical signals.

The rotation barrel 2330 is arranged outside the support barrel 2310 and may rotate around the support barrel 2310. The rotation barrel 2330 has a hollow structure in order for the support barrel 2310 to be inserted into the rotation barrel 2330. The rotation barrel 2330 may be connected to a driving unit 2114 installed on the housing 2100, for example, to a driving motor. The driving unit 2114 may be controlled by the controller 600 of the main body of the ultrasonic diagnostic apparatus 1000. As the driving unit 2114 rotates in one direction, the rotation barrel 2330 may rotate in one direction. Rotation velocity and a rotation direction of the rotation barrel 2330 may vary according to rotation velocity and a rotation direction of the driving unit 2114, and accordingly, rotation velocity and a rotation direction of the ultrasonic module 2300 supported by the rotation barrel 2330 may vary.

In the present embodiment, a case where the driving unit 2114 is arranged outside the rotation barrel 2330 is described, but a location and a shape of the driving unit 2114 may be appropriately changed according to necessity.

The rotation barrel 2330 supports the ultrasonic module 2300. The ultrasonic module 2300 and the rotation barrel 2330 may be fixed and supported by fixtures 2301. Since the ultrasonic module 2300 remains fixed to the rotation barrel 2330, the ultrasonic module 2300 rotates when the rotation barrel 2330 rotates.

The ultrasonic module 2300 includes contact electrodes 2302 formed to contact the ring electrodes 2312. The contact electrodes 2302 may transmit/receive electrical signals to/from the signal lines 2200 through the ring electrodes 2312. Materials of the contact electrodes 2302 may include an electrically conductive material so as to transmit the electrical signals to the ring electrodes 2312. A conductive material may be a metal such as copper, iron, aluminum, nickel, gold and silver. However, the conductive material is not limited thereto, and the contact electrodes 2302 may be formed of a non-conductive material which may be generally used to transmit electrical signals.

The contact electrodes 2302 pass through a hole 2331 formed in the rotation barrel 2330 and may be arranged to protrude towards the support barrel 2310 from an inside of the rotation barrel 2330.

Referring to FIGS. 5 and 6A, the contact electrodes 2302 protruding towards the support barrel 2310 contact the ring electrodes 2312 of the support barrel 2310, and the electrical signals are transmitted between them. A protrusion length (P) of each of the contact electrodes 2302 may be greater than a gap (G) between the ring electrodes 2312 and the inside of the rotation barrel 2330. An end portion of each contact electrode 2302 may be bent due to the contact with the ring electrodes 2312.

In this state, when the rotation barrel 2330 rotates around the support barrel 2310 in one direction, for example, a counterclockwise direction, the ultrasonic module 2300 supported by the rotation barrel 2330 rotates in the counterclockwise direction as shown in FIGS. 6B and 6C. The contact electrodes 2302 of the ultrasonic module 2300 rotate while remaining in contact with the ring electrodes 2312. Since each of the ring electrodes 2312 is continuously formed along the circumference direction of the support barrel 2310, the ring electrodes 2312 may remain in contact with the rotating contact electrodes 2302.

As described above, by forming the contact electrodes 2302 of the ultrasonic module 2300 and the ring electrodes 2200 connected to the signal lines 2200 as separate members, the signal lines 2200 may be fixed without rotation when the ultrasonic module 2300 rotates. As a result, the torsion or twists of the signal lines 2200, which occur when the ultrasonic module 2300 rotates in one direction, may be prevented.

While rotating, the ultrasonic module 2300 may be activated at some rotation regions. For example, as shown in FIG. 6A, entire rotation regions of the ultrasonic module 2300 may be classified into activation regions (ARs) where the ultrasonic module 2300 is relatively close to the target object 10 and non-activation regions (NARs) where the ultrasonic module 2300 is relatively far from the target object 10 as shown in FIGS. 6B and 6C. For example, a range of the activation regions (ARs) may be about 80° or smaller, and that of the non-activation regions (NARs) may exceed about 280°. The controller 600 may make the ultrasonic module 2300 transmit or receive the ultrasonic signals when the ultrasonic module 2300 is disposed at the activation regions (ARs). When the ultrasonic module 2300 is disposed at the non-activation regions (NARs), the controller 600 may prevent the ultrasonic module from transmitting or receiving the ultrasonic signals.

FIG. 7 is a cross-sectional view of an ultrasonic probe 2001 according to another embodiment of the present invention. FIG. 7 is a cross-sectional view showing parts of the ultrasonic probe 2001 of FIG. 3, taken along a lengthwise direction (L) of a housing 2100a of FIG. 3. The ultrasonic module 2300 may rotate about a rotation axis which has a direction parallel to the lengthwise direction (L) of the housing 2100a. Hereinafter, descriptions with regard to a configuration which is the same as that of FIG. 5 will be omitted, differences between two configurations will be described.

Referring to FIG. 7, the ultrasonic probe 2001 includes signal lines 2200, a support barrel 2310 in which the signal lines 2200 are arranged, a rotation barrel 2330 capable of rotating the support barrel 2310 and an ultrasonic module 2300 supported by the rotation barrel 2330.

The support barrel 2310 is arranged along a direction parallel to the lengthwise direction (L) of the housing 2100a. Ring electrodes 2312 are arranged outside or on the outer surface of the support barrel 2310 in order to be separated from each other in a lengthwise direction of the support barrel 2310. The lengthwise direction (L) of the housing 2100a may be parallel to that of the support barrel 2310. Each of the signal lines 2200 may be connected to each of the ring electrodes 2312.

Each of the ring electrodes 2312 is continuously formed along a circumference direction of support barrel 2310. Accordingly, the ring electrodes 2312 may remain in electrical contact with the ultrasonic module 2300 even though the ultrasonic module 2300 rotates in one direction. Materials of the ring electrodes 2312 may include an electrically conductive material so as to transmit electrical signals to the ultrasonic module 2300 and the signal lines 2200. A conductive material may be a metal such as copper, iron, aluminum, nickel, gold and silver. However, the conductive material is not limited thereto, and the ring electrodes 2312 may be formed of a non-conductive material which may be generally used to transmit electrical signals.

The rotation barrel 2330 is arranged outside the support barrel 2310 and may rotate around the support barrel 2310. The rotation barrel 2330 has a hollow structure in order for the support barrel 2310 to be inserted into the rotation barrel 2330. The rotation barrel 2330 may be connected to a driving unit 2114 installed on the housing 2100a. Therefore, the rotation barrel 2330 rotates due to the driving unit 2114.

The ultrasonic module 2300 and the rotation barrel 2330 may be fixed and supported by fixtures 2301. Since the ultrasonic module 2300 remains fixed to the rotation barrel 2330, the ultrasonic module 2300 and the rotation barrel 2330 rotate at the same time.

The ultrasonic module 2300 includes contact electrodes 2302 formed to contact the ring electrodes 2312. The contact electrodes 2302 may transmit/receive electrical signals to/from the signal lines 2200 through the ring electrodes. Materials of the contact electrodes 2302 may include an electrically conductive material so as to transmit the electrical signals to the ring electrodes 2312. A conductive material may be a metal such as copper, iron, aluminum, nickel, gold and silver. However, the conductive material is not limited thereto, and the contact electrodes 2302 may be formed of a non-conductive material which may be generally used to transmit electrical signals.

The contact electrodes 2302 pass through the rotation barrel 2330 and may be arranged to protrude towards the support barrel 2310 from an inside of the rotation barrel 2330. The contact electrodes 2302, which protrude towards the support barrel 2310, contact the ring electrodes 2312 of the support barrel 2310, and the electrical signals are transmitted between them.

When the rotation barrel 2330 rotates around the support barrel 2310 in one direction, the ultrasonic module 2300 supported by the rotation barrel 2330 may rotate in one direction. The contact electrodes 2302 of the ultrasonic module 2300 rotate while remaining in contact with the ring electrodes 2312. Since the ring electrodes 2312 are continuously formed along the circumference direction of the support barrel 2310, the ring electrodes 2312 may remain in contact with the rotating contact electrodes 2302.

As described above, by forming the contact electrodes 2302 of the ultrasonic module 2300 and the ring electrodes 2200 connected to the signal lines 2200 as separate members, the signal lines 2200 may be fixed without rotation when the ultrasonic module 2300 rotates. As a result, the torsion or twists of the signal lines 2200, which occur when the ultrasonic module 2300 rotates in one direction, may be prevented.

According to the one or more embodiments of the present invention, the ultrasonic probe and the ultrasonic diagnostic apparatus including the same include the ultrasonic module capable of rotating at least about 360° in one direction, and have a structure that prevent signal lines, which transmit signals, from being twisted despite rotation of the ultrasonic module. Thus, a speed of processing images may be improved, and also vibration and generation of noises may be prevented.

## Claims

1. An ultrasonic probe comprising:
a housing (2100);
an ultrasonic module (2300) arranged inside the housing (2100);
at least one signal line (2200) which transmits/receives signals to/from the ultrasonic module (2300);
a support barrel (2310) which is supported by the housing (2100), in which the at least one signal line (2200) is arranged and an outer surface of which has at least one ring electrode (2312) which electrically connects the at least one signal line (2200) to the ultrasonic module (2300); and
a rotation barrel (2330) which is rotatable around the support barrel (2310) in one direction and supports the ultrasonic module (2300) so as to maintain an electrical contact of the ultrasonic module (2300) to the at least one ring electrode (2312),
wherein the rotation barrel (2330) is arranged outside the support barrel (2310) and the ultrasonic module (2300) is supported on the rotation barrel (2330) by means of a plurality of fixtures (2301).

2. The ultrasonic probe of claim 1, wherein the ultrasonic module (2300) is formed to be rotatable at least about 360° in one direction due to the rotation barrel (2330).

3. The ultrasonic probe of claim 1, wherein the at least one ring electrode (2312) is continuously formed along a circumference direction of the support barrel (2310).

4. The ultrasonic probe of claim 1, wherein the at least one ring electrode (2312) comprises a plurality of ring electrodes, and the plurality of ring electrodes are arranged to be separated from each other along a lengthwise direction of the support barrel (2310).

5. The ultrasonic probe of claim 1, wherein the ultrasonic module (2300) further comprises contact electrodes (2302) protruding towards the support barrel (2310) from an inside of the rotation barrel (2330).

6. The ultrasonic probe of claim 5, wherein the contact electrodes (2302) are bent due to a contact with the at least one ring electrode (2312).

7. The ultrasonic probe of claim 1, wherein the ultrasonic probe further comprises a driving unit (2114) which rotates the rotation barrel (2330).

8. The ultrasonic probe of claim 1, wherein the ultrasonic module (2300) rotates about a rotation axis which is perpendicular to a lengthwise direction of the housing (2100).

9. The ultrasonic probe of claim 8, wherein the ultrasonic module (2300) further comprises a controller (600) which makes the ultrasonic module (2300) transmit or receive ultrasonic signals at some of regions where the ultrasonic module (2300) rotates while the ultrasonic module (2300) rotates.

10. The ultrasonic probe of claim 1, wherein the ultrasonic module (2300) rotates about a rotation axis which is parallel to lengthwise direction of the housing (2100).

11. An ultrasonic diagnostic apparatus comprising:
an ultrasonic probe (2000) of any preceding claim;
an image processor (200) which generates an ultrasonic image based on the received echo signal; and
a display unit (230) which displays the generated ultrasonic image.

## Patentansprüche

1. Ultraschallsonde, welche Folgendes aufweist:
ein Gehäuse (2100);
ein Ultraschallmodul (2300), das innerhalb des Gehäuses (2100) angeordnet ist;
wenigstens eine Signalleitung (2200), die Signale zu/von dem Ultraschallmodul (2300) überträgt/empfängt;
eine durch das Gehäuse (2100) gehaltene Stütztrommel (2310), in der die wenigstens eine Signalleitung (2200) angeordnet ist und von der eine äußere Fläche wenigstens eine Ringelektrode (2312) aufweist, die die wenigstens eine Signalleitung (2200) mit dem Ultraschallmodul (2300) elektrisch verbindet; und
eine Rotationstrommel (2330), die um die Stütztrommel (2310) in einer Richtung rotierbar ist und das Ultraschallmodul (2300) abstützt, um einen elektrischen Kontakt des Ultraschallmoduls (2300) zu der wenigstens einen Ringelektrode (2312) aufrecht zu erhalten,
wobei die Rotationstrommel (2330) außerhalb der Stütztrommel (2310) angeordnet ist und das Ultraschallmodul (2300) auf der Rotationstrommel (2330) mittels einer Vielzahl von Befestigungselementen (2301) gehalten ist.

2. Ultraschallsonde nach Anspruch 1, wobei das Ultraschallmodul (2300) so ausgebildet ist, dass es aufgrund der Rotationstrommel (2330) um wenigstens 360° in einer Richtung rotierbar ist.

3. Ultraschallsonde nach Anspruch 1, wobei die wenigstens eine Ringelektrode (2312) kontinuierlich entlang einer Umfangsrichtung der Stütztrommel (2310) gebildet ist.

4. Ultraschallsonde nach Anspruch 1, wobei die wenigstens eine Ringelektrode (2312) eine Vielzahl von Ringelektroden aufweist, und die Vielzahl von Ringelektroden so angeordnet sind, dass sie in einer Längsrichtung der Stütztrommel (2310) voneinander getrennt werden können.

5. Ultraschallsonde nach Anspruch 1, wobei das Ultraschallmodul (2300) des Weiteren Kontaktelektroden (2302) aufweist, die von innerhalb der Rotationstrommel (2330) in Richtung der Stütztrommel (2310) überstehen.

6. Ultraschallsonde nach Anspruch 5, wobei die Kontaktelektroden (2302) aufgrund eines Kontakts mit der wenigstens einen Ringelektrode (2312) gebogen sind.

7. Ultraschallsonde nach Anspruch 1, wobei die Ultraschallsonde des Weiteren eine Antriebseinheit (2114) aufweist, welche die Rotationstrommel (2330) rotiert.

8. Ultraschallsonde nach Anspruch 1, wobei das Ultraschallmodul (2300) um eine Rotationsachse rotiert, die senkrecht zu einer Längsrichtung des Gehäuses (2100) ist.

9. Ultraschallsonde nach Anspruch 8, wobei das Ultraschallmodul (2300) des Weiteren ein Steuergerät (600) aufweist, welches das Ultraschallmodul (2300) dazu bringt, Ultraschallsignale in einigen Bereichen, in denen das Ultraschallmodul (2300) rotiert, zu übertragen oder zu empfangen, während das Ultraschallmodul (2300) rotiert.

10. Ultraschallsonde nach Anspruch 1, wobei das Ultraschallmodul (2300) um eine Rotationsachse rotiert, die parallel zu der Längsrichtung des Gehäuses (2100) ist.

11. Ultraschalldiagnosevorrichtung, welches Folgendes aufweist:
eine Ultraschallsonde (200) nach einem der vorhergehenden Ansprüche;
eine Bildverarbeitungseinheit (200), die ein Ultraschallbild basierend auf dem empfangenen Echosignal erzeugt; und
eine Anzeigeeinheit (230), die das erzeugte Ultraschallbild anzeigt.

## Revendications

1. Sonde ultrasonique comprenant :
un boîtier (2100) ;
un module ultrasonique (2300) agencé à l'intérieur du boîtier (2100) ;
au moins une ligne de signal (2200) qui transmet/reçoit des signaux au/du module ultrasonique (2300) ;
un cylindre de support (2310) qui est supporté par le boîtier (2100), dans laquelle ladite au moins une ligne de signal (2200) est agencée et dont la surface extérieure présente au moins une électrode annulaire (2312) qui relie électriquement ladite au moins une ligne de signal (2200) vers le module ultrasonique (2300) ; et
un cylindre de rotation (2330) pouvant tourner autour du cylindre de support (2310) dans une direction et supportant le module ultrasonique (2300) de manière à maintenir un contact électrique du module ultrasonique (2300) avec ladite au moins une électrode annulaire (2312),
dans laquelle le cylindre de rotation (2330) est agencé à l'extérieur du cylindre de support (2310) et le module ultrasonique (2300) est supporté sur le cylindre de rotation (2330) au moyen d'une pluralité de fixations (2301).

2. Sonde ultrasonique selon la revendication 1, dans laquelle le module ultrasonique (2300) est formé pour pouvoir tourner sur au moins environ 360° dans un sens en raison du cylindre de rotation (2330).

3. Sonde ultrasonique selon la revendication 1, dans laquelle ladite au moins une électrode annulaire (2312) est formée en continu le long d'une direction de circonférence du cylindre de support (2310).

4. Sonde ultrasonique selon la revendication 1, dans laquelle ladite au moins une électrode annulaire (2312) comprend une pluralité d'électrodes annulaires, et la pluralité d'électrodes annulaires est agencée pour être séparées les unes des autres le long d'une direction longitudinale du cylindre de support (2310).

5. Sonde ultrasonique selon la revendication 1, dans laquelle le module ultrasonique (2300) comprend en outre des électrodes de contact (2302) faisant saillie vers le cylindre de support (2310) depuis l'intérieur du cylindre de rotation (2330).

6. Sonde ultrasonique selon la revendication 5, dans laquelle les électrodes de contact (2302) sont pliées en raison d'un contact avec ladite au moins une électrode annulaire (2312).

7. Sonde ultrasonique selon la revendication 1, dans laquelle la sonde ultrasonique comprend en outre une unité d'entraînement (2114) qui fait tourner le cylindre de rotation (2330).

8. Sonde ultrasonique selon la revendication 1, dans laquelle le module ultrasonique (2300) tourne autour d'un axe de rotation qui est perpendiculaire à une direction longitudinale du boîtier (2100).

9. Sonde ultrasonique selon la revendication 8, dans laquelle le module ultrasonique (2300) comprend en outre une commande (600) qui fait en sorte que le module ultrasonique (2300) transmette ou reçoive des signaux ultrasoniques dans certaines des régions où le module ultrasonique (2300) tourne pendant que le module ultrasonique (2300) tourne.

10. Sonde ultrasonique selon la revendication 1, dans laquelle le module ultrasonique (2300) tourne autour d'un axe de rotation qui est parallèle à une direction longitudinale du boîtier (2100).

11. Appareil de diagnostic ultrasonique comprenant :
une sonde ultrasonique (2000) selon l'une quelconque des revendications précédentes ;
un processeur d'image (200) qui génère une image ultrasonique sur la base des signaux d'écho reçus ; et
une unité d'affichage (230) qui affiche l'image ultrasonique générée.
